# EUROPEAN PATENT SPECIFICATION

(11) **EP 2 956 204 B1**
(45) Date of publication and mention of the grant of the patent: **28.08.2019**
(21) Application number: 14709349.6
(22) Date of filing: 17.02.2014
(51) Int. Cl.: A61M 39/10, A61M 39/12, A61M 39/24, A61M 39/26

(54) **RELEASABLE CONNECTION FOR A TUBE**
LÖSBARE VERBINDUNG FÜR EIN ROHR
RACCORDEMENT AMOVIBLE POUR UN TUBE

(30) Priority: 18.02.2013 GB 201302765
(43) Date of publication of application: 23.12.2015
(73) Proprietor: Salisbury NHS Foundation Trust, Salisbury, Wiltshire SP2 8BJ (GB)
(72) Inventor: LAUGHTON, Richard, Salisbury Wiltshire SP2 8BJ (GB)
(74) Representative: Akers, Noel James
(86) International application number: PCT/GB2014/000056
(87) International publication number: WO 2014/125245

(56) References cited:
- EP-A2- 0 795 342
- WO-A1-2013/076667
- US-A- 2 926 934
- US-A- 5 492 147
- US-A1- 2010 230 950
- US-A1- 2012 042 971
- US-A1- 2013 030 387

## Description

The present invention relates to a releasable connection for a tube or a line. The invention finds particular use in the protection of medical tubes and lines, such as intravenous lines, catheters and the like.

Many medical conditions require a tube or line to be provided to a patient. Generally, such tubes or lines are used to provide fluid to a patient or for the removal of fluid from the patient. Examples of such tubes and lines include cannulas, such as intravenous (IV) cannulas, catheters, drain lines, epidural lines, nasogastric tubes, percutaneous endoscopic gastrostomy (PEG) lines, central lines, peripherally inserted central catheter (PICC) lines and the like. For the sake of brevity, all such tubes and lines will be referred to hereinafter generally as 'lines'.

Lines provided to a patient are vulnerable to being dislodged or even pulled out by the line being pulled or tugged. Such pulls or tugs may arise, for example, as a result of movement of the patient themselves or the movement of others. In some circumstances, disturbed patients may be moved to attempt to dislodge or remove lines themselves. It will be appreciated that the dislodging or inadvertent removal of a line from a patient may result in severe injury to the patient or, in extreme cases, death. In many cases, for example when a cannula is inadvertently pulled out of a patient, the entire fluid delivery system is treated as being compromised and is replaced to ensure sterility is maintained and to avoid any risk of infection.

Further, depending upon the nature of the treatment being undergone by the patient, a broken or dislodged line could result in the spillage of liquids. This is particularly serious in cases where the liquid spilled is harmful, for example toxic, as in the case of a line being used to administer a chemotherapeutic agent. It will also be appreciated that any spillage of a drug or other agent being administered to a patient prevents the dose received by the patient from being accurately determined.

Clearly, there is a need to protect the patients and medical staff from the inadvertent dislodging or removal of lines. One approach is to firmly secure the lines to the patient, such that a significant force is required to move or remove the line. This approach is routinely taken in many cases. For example, it is known to secure drain lines to the patient by means of sutures. Further, it is known to firmly secure lines, such as IV cannulas, to patients using adhesive tape. While this approach may protect against light tugs and pulls on the lines, injury may still arise from tugs or pulls that apply a significant force to the line, such as the patient tripping or falling.

Another approach to this problem is to provide means by which the line is broken or separated in the event of a large enough tug or pull. Such devices are known in the art.

For example, GB 2,343,723 discloses a medical fluid line arrangement, in which two coupling elements are coupled together and separable by a predetermined separating force applied to the elements. Each coupling element is applied to the end of a respective tube. The force required to separate the elements when coupled may be varied.

US 3,951,153 concerns a safety device for a catheter or the like, such as a Foley catheter. The safety device includes a coupler assembly. The catheter is cut and a plurality of slits made in one of the severed ends to form a plurality of strips. A hollow tubular projection of the coupler is fitted into the opening of the slit end, the projection having notches to accommodate the strips. A second hollow tubular projection of the coupler is fitted to the other of the severed ends.

A skin mounted drainage catheter retention disc is shown and described in US 4,533,349. A connecting tube having a low release force connector at either end is secured to the disc and links the catheter to a drainage bag.

US 5,405,336 discloses a connector for a catheter system. The connector comprises two sections are joined together to have a predetermined frictional engagement, such that the sections release if a force sufficient to overcome the friction is applied to the catheter. The sections may be provided with a sheath therearound, to maintain the sterility of the catheter at the junction of the two sections and in the event the sections are separated.

A disconnect assembly for medical access devices is described and shown in US 5,820,614. The assembly comprises a first portion having a first valve and a second portion having a second valve therein. The two portions are releasably connected. The first and second valves are each biased to a normally closed position. When the two portions are connected, the first and second valves are held open against the bias. Applying a sufficiently high force to the connection disconnects the first and second portions, allowing the first and second valves to close.

US 2005/0015075 discloses a coupling device for medical lines. The device has two parts, each having a valve and being coupled to a medical line. The parts connect together by a snap fit mechanism having a rounded protrusion and rounded groove arrangement, that pulls apart under a sufficient force. When pulled apart, the valves seal their respective medical lines.

US 2007/02444468 concerns a safety device for use with catheters. The device has a flexible, pleated section that permits the catheter to be axially elongated when a force is applied. In one embodiment, the device comprises a fluid tight connector that connects two portions of the catheter and allows rotational movement of one portion with respect to the other. The device does not permit the catheter to be separated under the application of excessive force, for example a force exceeding that required to fully elongate the pleated section.

A break-away hemostasis hub for a catheter or introducer sheath is shown and described in US 2008/0108976, having a splittable or separable housing and a seal therein.

US 2008/0197626 discloses a coupling device for medical lines. The device has two parts, each coupled to a respective line. One of the parts provides both a detachable coupling having a breakaway connection, operable to separate when subjected to a sufficient separation force, and a secure locking mechanism that requires manual separation.

A breakaway percutaneous endoscopic gastronomy (PEG) tube is disclosed in US 2011/0112482. The peg tube has a releasable connector between two tube segments. The connector separates under the action of sufficient force, to prevent the PEG tube being pulled inadvertently from the abdomen of the patient.

WO 2010/069361 discloses a connection arrangement and method for connecting a medical device to the connection arrangement. The arrangement comprises a rupterable retaining member, arranged to rupture at a predetermined breaking force, thereby allowing the connection to be separated.

More recently, WO 2013/076667 discloses a connector for medical lines. The connector comprises first and second portions and has first and second valves. The connector further comprises a central duct disposed between the first and second portions. When a pulling force exceeding a predetermined threshold value is applied, the central duct detaches, allowing at least one of the valves to close.

US 2013/030387 discloses a coupling for medical fluids.

EP 0 795 342 discloses a disconnect for medical access devices.

There is a need for an improved device for protecting medical lines and patients from pulls and tugs on the lines. It would be advantageous if the device could be simple to construct and operate. Preferably, the device should maintain the sterility of the medical line and require the minimum of sterilisation to be installed or replaced.

According to a first aspect of the present invention there is provided a coupling assembly for use in a coupling device, the coupling assembly having a conduit therethrough and being for releasably coupling to both of a first valve assembly and a second valve assembly, the coupling assembly when so coupled opening both the first and second valves to allow fluid to flow from the first line segment through the coupling assembly to the second line segment or medical device;
characterised in that
the coupling assembly further comprises releasable coupling means to releasably hold the coupling assembly coupled to both the first and second valve assemblies, the coupling means releasing the coupling assembly from both the first and second valve assemblies when the first line segment and the medical device or the first and second line segments are subjected to a sufficiently high separation force;
wherein the coupling means comprises one or more resilient coupling members, the or each coupling member being permanently attached to the coupling assembly; wherein the or each coupling member comprises a spring clip, wherein the spring clip is permanently attached to the coupling assembly at a central portion and has opposing first and second end portions to releasably engage with a respective one of the first and second valve assemblies.

According to the present invention, there is also provided a releasable coupling device for a medical line, the device comprising:
a first valve assembly comprising a first valve biased to a closed position, the first valve assembly for attaching to the end portion of a line segment;
a second valve assembly comprising a second valve biased to a closed position, the second valve assembly for attaching to a medical device; and
a coupling assembly as hereinbefore defined.

The device of the present invention is a releasable device for inclusion in a medical line. The components of the device are arranged to separate in the event the line is subjected to a threshold force, for example by a pull or tug being applied to the line. Further, once separated, the device acts to seal the ends of the medical line on each side of the device, thereby preventing the loss of any fluid from the lines or, perhaps more importantly, maintaining the lines sterile. In this way, in the event of the device separating, the medical line is not compromised and it is not necessary to replace the lines. Rather, as described in more detail below, the device comprises a minimum number of components that require replacement or resterilisation.

Further, in the case of intravenous fluids or the like being administered with a pump, the resulting back-pressure from the sealed line arising from the operation of the device will typically cause the pump to alarm, thus alerting the practitioner.

Another advantage of the device of the invention is that it is simple to manufacture at a low cost, as it can employ components known and available commercially.

The coupling device of the present invention may be used in systems for the delivery of fluid to a patient, such as the delivery of a drug or other agent, for example during chemotherapy, or may be used in systems for removing fluid from a patient, such as a drain line or a catheter system.

The device of the present invention comprises first and second valve assemblies. In use, the first valve assembly is attached to the end of a segment of the medical line, to allow the flow of fluid therealong. The second valve assembly is connected to a medical device. This may be the end portion of a second segment of the medical line. Alternatively, the second valve assembly may be connected to another medical device, for example to a connector of a liquid reservoir, such as a bag of fluid to be delivered intravenously, or the proximal end of the catheter hub of an IV catheter assembly.

Accordingly, each valve assembly comprises a fitting to secure the assembly to the end of a segment of medical line or the medical device. Fittings for securing each valve assembly to the end portion of the segment of the medical line and the medical device are known in the art and include threaded fittings, push fittings, bayonet fittings and the like. Many fittings employ a standard fitting, known in the art as a Luer taper, which typically comprises a male member or nozzle having a standard Luer taper on its outside, to be received within a female Luer taper.

Each of the first and second valve assemblies comprises a conduit therethrough for the flow of fluid and a valve. The function of the valve is to close the conduit, thus closing the respective line segment or device to which the valve assembly is attached and providing a fluid-tight seal to prevent fluid entering or leaving the line. Each valve is biased to the closed position, that is the position in which the line or device is sealed. Any suitable valve arrangement may be used. Suitable valve arrangements are known in the art. The valves of the first and second valve assemblies may be the same or different. Preferably, the valves of the first and second valve assemblies are the same.

One suitable valve is a diaphragm or membrane valve, that is a valve comprising a flexible membrane extending across the conduit. The membrane is provided with one or more slits therein and is inherently biased to the closed position. Such a valve is typically opened by an actuator, that is a member forced against the diaphragm or membrane, causing it to flex and open.

A preferred valve arrangement is one that is activated, in particular opened, by the insertion of a projection, nozzle or spigot, for example a male Luer. Such valve arrangements are known in the art and referred to in some cases as 'Luer-activated' valves. Examples of such valves are contained in the devices disclosed in US 5,535,785; US 5,775,671; WO 97/24548; WO 2008/048776; US 2008/172005; and US 6,039,302. In general, each of these valve assemblies comprises a valve biased to the closed position and opened upon insertion of the projection, nozzle or spigot, in particular a nozzle having a male Luer taper. One preferred arrangement of a Luer-activated valve is available commercially as the Posiflow™ device from Becton Dickinson.

In use, each of the first and second valve assemblies is attached to the end portion of a segment of the medical line or a medical device. The first and second valves are held in the open position by the coupling assembly, details of which are described below. When subjected to a sufficient force, for example from a tug or pull on the medical line, the coupling assembly is released from both the first and second valve assemblies, allowing the valves to close under the action of their inherent or inbuilt biases, thereby sealing the line segments or device against fluid ingress or egress.

The device of the present invention further comprises a coupling assembly having a conduit therethrough for the flow of fluid. The coupling member is for releasably coupling to both of the first valve assembly and the second valve assembly. As noted above, the coupling assembly when so coupled opens both the first and second valves to allow fluid to flow from the first line segment through the coupling assembly to the medical device or a second line segment.

The coupling assembly comprises means to open both the first and the second valves in each of the first and second valve assemblies. In one embodiment, the coupling assembly comprises a first valve actuator and a second valve actuator, each of the valve actuators respectively opening the first and second valves when the coupling assembly is connected to the first and second valve assemblies. The first and second valve actuators are arranged to cooperate with the respective valve assembly and may be the same or different. Preferably, the first and second valve actuators are the same. In a preferred embodiment, the first and second valve actuators are disposed at opposite ends of the coupling assembly.

A preferred valve actuator comprises a projection, nozzle or spigot extending from the coupling assembly, in particular the projection, nozzle or spigot being operable to open the valve. More preferably, the nozzle is in the form of a male Luer taper, in particular to cooperate with a Luer-activated valve arrangement, as described above.

The coupling assembly, when connected to the first and second valve assemblies is sealed thereto, to prevent the leakage of fluid into or out of the assembly and the medical line or device. Any form of seal arrangement may be employed to seal the coupling assembly to each valve assembly. In particular, the coupling assembly may cooperate with the valve arrangement within the valve assembly to provide the seal. In embodiments in which the coupling assembly comprises a valve actuator, the valve actuator may seal with the respective valve assembly. This is particularly the case when a Luer taper is employed, as is known in the art. For example, the valve actuator, such as a Luer taper, may seal against a membrane of the valve assembly.

As described above, the coupling assembly is released from both the first and second valve assemblies in the event of being subjected to sufficient force. It is important to have the coupling assembly release from both valve assemblies, in order to have both the first and second valves close and seal the ends of the medical line segments or the medical device to which the valve assembly is connected. Accordingly, it is particularly preferred to have the coupling assembly biased out of engagement with and away from at least one, more preferably both, of the first and second valve assemblies. In some embodiments, the bias inherent in the valve assembly to close the valve may be relied upon to release the coupling assembly from the valve assembly. Alternatively, it may be preferred to provide means to bias the coupling assembly away from each of the first and second valve assemblies, to facilitate the release of the coupling assembly from both valve assemblies. The means to bias the coupling assembly away from each of the first and second valve assemblies is preferably provided in the coupling assembly. Suitable means include one or more resilient means to urge the coupling assembly away from each valve assembly. In one preferred arrangement, the resilient means is compressed under the action of the coupling assembly being connected to the valve assembly, thereby generating the force biasing the coupling assembly and the valve assembly apart. Suitable resilient means include a spring, such as a coil spring. More preferably, the biasing means comprises a resilient material, for example an elastomeric body. In one embodiment, the coupling assembly comprises a resilient means extending around each valve actuator, for example a ring of an elastomer extending around the projection, nozzle or spigot.

The device of the present invention further comprises releasable coupling means to releasably hold the coupling assembly coupled to both the first and second valve assemblies. The coupling means is arranged to release the coupling assembly from both the first and second valve assemblies when the first and second line segments are subjected to a sufficiently high separation force.

The coupling means may be a direct action means, that is the coupling means releases under the action of a force applied longitudinally along the medical line and passing longitudinally through the device. Alternatively, the coupling means may be an indirect action means, that is responds to a force applied to the medical line which is not directed longitudinally through the device. The device may comprise both direct and indirect action coupling means.

The coupling means comprises one or more resilient coupling members. The or each coupling member is permanently attached to the coupling assembly and releasably coupling the coupling assembly to at least one of the valve assemblies.

The resilient coupling member comprises a spring clip, permanently attached to the coupling assembly and releasably coupling to a valve assembly, the spring clip being resilient and biased to hold the coupling assembly connected to the valve assembly, until subjected to sufficient force to overcome the resilient bias of the clip and release the coupling assembly from the valve assembly. The spring clip is permanently attached to the coupling assembly at a central portion and has opposing first and second end portions to releasably engage with a respective one of the first and second valve assemblies. The spring clip is preferably pivotally mounted to the coupling assembly. In this way, the release of the valve assembly at one end of the spring clip reduces the coupling force at the other end of the spring clip, in turn releasing the coupling assembly from the other valve assembly. Preferably, the coupling means comprises a plurality of spring clips, more preferably arranged around the circumference of both the coupling assembly and the valve assembly.

In one embodiment of an indirect action coupling means, the coupling means comprises one or more coupling members. The or each coupling member holds the coupling assembly in engagement with one, more preferably both, of the valve assemblies. A force on the line is arranged to act directly on the coupling member, in turn releasing the coupling assembly from engagement with at least one of the valve assemblies, more preferably both valve assemblies.

The coupling member may be attached to at least one valve assembly and to the coupling assembly. More preferably, the coupling member is attached to both the first and second valve assemblies. The coupling member is releasably attached to one or both of the valve assemblies. The coupling member may be substantially rigid, but is more preferably flexible. In particular, the coupling member is preferably sufficiently flexible to be able to bend under the action of a force applied to the medical line. The coupling member is attached to a portion of the medical line spaced from the coupling device, so that a force applied to the line spaced from the coupling device is transmitted to the coupling member. The coupling member is attached to the portion of the medical line either directly, for example by having the said portion of the line mounted on the coupling member by a clip or the like, or indirectly, for example by a tether connected to and extending between the coupling member and the said portion of the medical line. In use, a force applied to the portion of the line spaced from the coupling device acts on the coupling member, releasing it from at least one of the assemblies to which it is attached, thereby releasing the coupling assembly. In a preferred arrangement, the coupling member is attached to both the first and second valve assemblies, being releasably attached to at least one thereof. In this way, the coupling member holds the first and second valve assemblies in engagement with the coupling assembly, such that releasing the coupling member from one of the first and second valve assemblies causes the coupling assembly to be released from both.

The force required to release the coupling means and uncouple the coupling assembly from the first and second valve assemblies may vary from embodiment to embodiment, depending upon the nature and use of the line in which the coupling device is to be installed. Preferably, the force required to release the coupling device is from 0.5 to 20 N, more preferably from 1 to 10 N, still more preferably from 2 to 6 N.

The components of the coupling device arid/or their packaging may be marked to indicate the rating of the device, in terms of the force required to uncouple the components in the event of a pull or tug on the medical line. For example, some or all of the components may be colour coded.

The coupling device of the present invention may be provided with the component parts separated, ready to be assembled and installed by the user. Alternatively, the components may be assembled, with the first and second valve assemblies connected to the coupling assembly and ready for installation in a line. In this case, the entire assembly may be primed with a suitably sterilised liquid, for example saline or sterilised water. The liquid will remain in the conduits in the coupling device while the device is being installed, and will assist in preventing air entering the line or the patient. As a further alternative, the assembled coupling device may be connected to the line and primed with fluid as the line itself is primed.

The coupling device may be provided with suitable adaptors, such as a Luer female to female adaptor or the like, to allow the valve assemblies to be connected with other fittings being used, such as a catheter hub or the like.

In operation, the coupling device of the present invention is installed with the first valve assembly connected to the end portion of a segment of the medical line. The second valve assembly is connected to a medical device or to the end portion of a second segment of the medical line. In this condition, both the first and second valves are closed and the medical line and device are fluid tight. The coupling assembly is coupled to the first valve assembly and the second valve assembly and held in the coupled position by the coupling means. The action of connecting the coupling assembly to each valve assembly opens both the first and second valves, allowing for fluid to flow between the medical line and the medical device through the coupling device. It may be preferred to fill the conduit in the coupling assembly with a suitable liquid, for example saline solution, to prevent an air bubble being present in the system, which may possible enter the patient. Due to the size of the conduit, the liquid will generally remain in the coupling assembly by capillary action, allowing the coupling assembly to be engaged with both the first and second valve assemblies and the coupling means engaged.

The medical line and device are used in the conventional manner. Should sufficient force be applied to the medical line, the coupling means is caused to release the coupling between the coupling assembly and at least one of the valve assemblies. The action of the coupling assembly being biased away from both valve assemblies causes the coupling assembly to be released from the other valve assembly. As a result of this release, both the first and second valves are caused to close, thereby sealing the end of the medical line segment and the medical device or other line segment, preventing contamination of either the line or the medical device and preventing the spillage of any fluid.

The coupling device of the present invention may then be reconnected by repeating the installation procedure described above. Typically, it will only be necessary to sterilise the exposed ends of the first and second valve assemblies, for example with a suitable sterile wipe, or the like. The coupling assembly may be resterilised, before being used again. More preferably, a sterile replacement coupling assembly is used to re-establish the connection. In this respect, the coupling assembly may be considered to be a disposable item, replaced each time the coupling device is reassembled after a release. It is an advantage of the coupling device of the present invention that the other components, such as the valve assemblies, due to their construction, do not need replacing and function to prevent the fluid system being compromised and rendered unsterile. The coupling assembly is typically primed with a sterile fluid, such as saline, before the coupling device is reassembled in the line.

As noted above, the coupling assembly of the coupling device may be treated as a disposable item. As a result, a single coupling device, when in use, may employ two or more coupling assemblies, depending upon the number of inadvertent releases that occur.

As the coupling assembly, when released, may fall from the device, the coupling assembly may be connected to another component, such as one of the valve assemblies, for example by a tether, allowing for swift and easy location and retrieval of the coupling assembly. However, any such connection should not compromise the action of the coupling device, in particular prevent the coupling assembly from being released from both valve assemblies and allowing the valve in each valve assembly to close.

Embodiments of the present invention will now be described, by way of example only, having reference to the accompanying drawings, in which:
Figure 1 is a view of a medical device installation comprising an IV catheter, a medical line and a coupling device according to a first embodiment;
Figure 2 is an exploded view of the components of the coupling device of the embodiment of Figure 1;
Figure 3 is a cross-sectional view of a coupling assembly according to one embodiment for use in the coupling device of Figures 1 and 2;
Figure 4 is a view of a medical device installation comprising a medical line and a coupling device according to a second embodiment of the present invention;
Figure 5 is an exploded view of the components of the coupling device of Figure 4;
Figure 6 is a cross-sectional view of a coupling assembly according to a further embodiment of the present invention for use in the coupling device of Figures 4 and 5;
Figure 7 is a cross-sectional view of a coupling assembly engaged with first and second valve assemblies, showing the valves of the valve assemblies in the open position; and
Figure 8 is a cross-sectional view of the coupling assembly and valve assemblies of Figure 7 in a disengaged condition, showing the valves of the valve assemblies in the closed position.

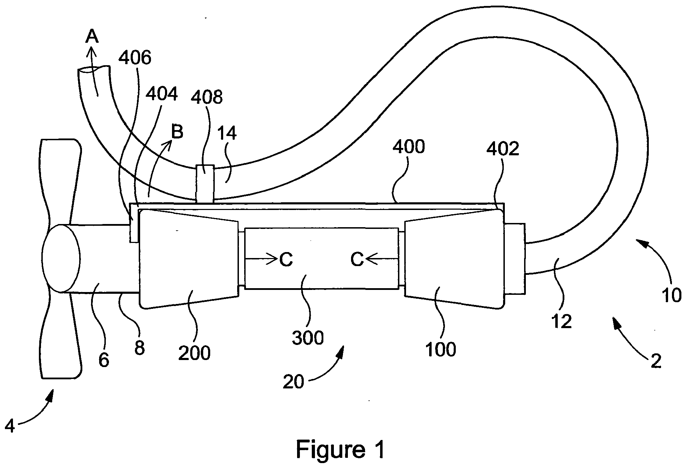

Turning to Figure 1, there is shown a medical device installation, generally indicated as 2. The installation comprises an IV catheter assembly 4 having a configuration well known in the art and commercially available. The IV catheter assembly 4 comprises a catheter hub 6 having, at its proximal end 8, a standard female Luer taper.

The first embodiment and figures 1, 2, and 3 are not part of the invention.

The installation 2 further comprises a medical line 10, having an end portion 12. The medical line 10 is connected to a reservoir of liquid (not shown for clarity) to be administered to a patient in a conventional manner.

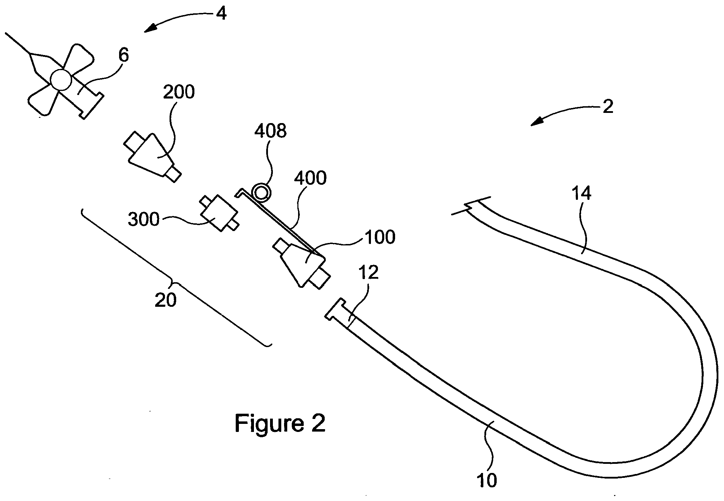

A coupling device 20 according to one embodiment is used to connect the medical line 10 to the proximal end 8 of the catheter hub 6 of the IV catheter assembly 4. The components of the coupling device 20 are shown in an exploded view in Figure 2.

Referring to Figure 2, the coupling device 20 comprises a first valve assembly 100 permanently secured to the end portion 12 of the medical line 10, again in known manner. Fittings for securing the first valve assembly to the medical line 10 are known in the art, for example female to female Luer adapters and the like (not shown for clarity). The coupling device further comprises a second valve assembly 200 permanently attached to the female Luer taper 8 of the catheter hub 6 of the IV catheter assembly 4, in conventional manner. In this respect, a permanent attachment is one that is not liable to release when subjected to a force applied to the medical line 10.

Each of the first and second valve assemblies 100, 200 comprises a valve, as described hereinafter, which is biased into the closed position, in which the valve seals the valve assembly and prevents the flow of fluid therethrough. When coupled to the coupling assembly 300, as shown in Figure 1, the action of the coupling assembly is to open the valve in each valve assembly 100, 200, allowing the flow of fluid therethrough.

It is to be understood that the coupling device 20 is shown in Figure 1 attached by way of the second valve assembly 200 to the catheter hub of an IV catheter assembly, by way of example only. The second valve assembly 200 may be connected in like manner to another medical device, for example another segment of medical line and the coupling device 20 will operate in the same manner as described herein.

The coupling device 20 further comprises a coupling assembly 300 disposed between the first and second valve assembly 100, 200 and releasably coupled to each of the first and second valve assemblies. The coupling assembly 300 is biased away from and out of engagement with each of the first and second valve assemblies 100, 200. The coupling assembly 300 is held engaged with both the first and second valve assemblies 100, 200 by a coupling member 400. The coupling member 400 is permanently mounted at one end 402 to the first valve assembly 100. The second end 404 of the coupling member 400 is provided with arm 406 for releasably engaging with and gripping the body of the second valve assembly 200, as shown in Figure 1. As shown, with the coupling member 400 releasably engaged with the second valve assembly 200, the coupling assembly 300 is held between the first and second valve assemblies 100, 200 and engaged therewith, such that the first and second valves are held open and fluid can flow through the device.

A clip 408 is provided on the coupling member 400 adjacent the second end 404 and holds a portion 14 of the medical line 10 spaced from the end portion 12, also as shown in Figure 1. The clip 408 is split, to allow the line 10 to be inserted and removed, as required.

The components of the coupling device are shown in the disassembled condition in Figure 2, from which the components are assembled into the configuration shown in Figure 1, in which configuration the coupling device is used and allows fluid to flow from the medical line 10 through the coupling device 20 into the IV catheter 4, in conventional manner.

Referring again to Figure 1, the coupling member 400 is of a resilient material, such that a force applied to the medical line 10, indicated by the arrow A, for example from the line being inadvertently tugged or pulled sharply, causes the coupling member 400 to bend, such that the second end 404 moves in the direction of the arrow B, releasing the arm 406 from engagement with the second valve assembly 200. As a result of the arm 406 releasing from the second valve assembly 200, the coupling assembly 300 is no longer held between and connected to the valve assemblies 100 and 200. The biasing forces acting on the coupling assembly 300 are indicated by the arrows C, the action of which urges the coupling assembly away from and out of connection with both valve assemblies 100, 200. The release of the coupling assembly 300 from both valve assemblies 100, 200 allows the valve in each valve assembly to close.

As discussed, the arrangement shown in Figure 1 has the portion 14 of the medical line 10 directly connected to the coupling member 400 by way of the clip 408. An alternative arrangement is to have the said portion 14 connected to the clip 408 by way of a tether, such that a pull on the medical line 10 is transferred by the tether to the coupling member 400, causing the coupling member to release from the second valve assembly 200.

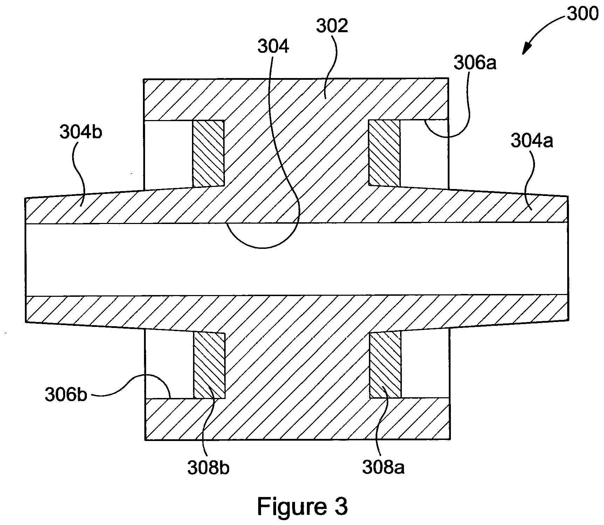

Turning to Figure 3, there is shown a cross-sectional view of the coupling assembly 300 for use in the coupling device of Figures 1 and 2. The coupling assembly 300 has a generally cylindrical body 302 having a generally cylindrical conduit 304 extending therethrough and having end portions forming nozzles 304a and 304b. Each of the nozzles 304a and 304b of the conduit extends beyond the body 302 and is formed with a Luer taper on its outer surface, to form a Luer male member, as is known in the art. Each nozzle 304a and 304b functions as a valve actuator for the respective valve assembly 100, 200, as described in more detail below.

As noted above, the coupling assembly 300 is biased away from and out of engagement with each of the valve assemblies 100, 200. This bias may be provided solely by the valve in the valve assembly, as described in more detail below. In the embodiment shown in Figure 3, the body 302 of the coupling assembly 300 is provided with an annular groove 306a, 306b around each nozzle 304a, 304b. An elastomeric ring 308a, 308b is provided in each annular groove. When the coupling assembly 300 is coupled to the valve assemblies 100, 200, each elastomeric ring 308a, 308b is compressed and provides a force biasing the coupling assembly 300 away from each valve assembly.

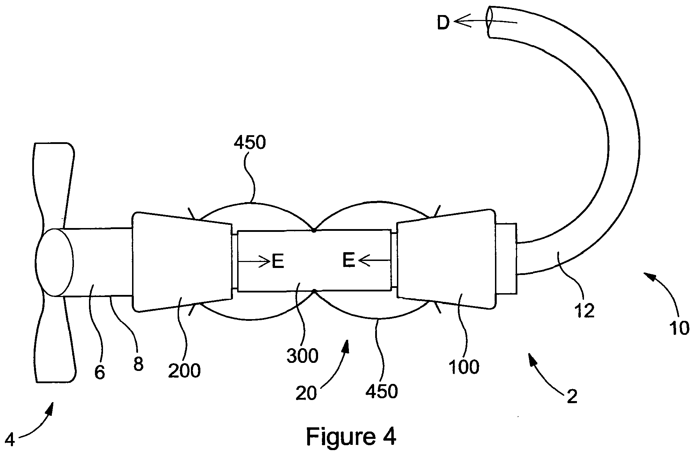

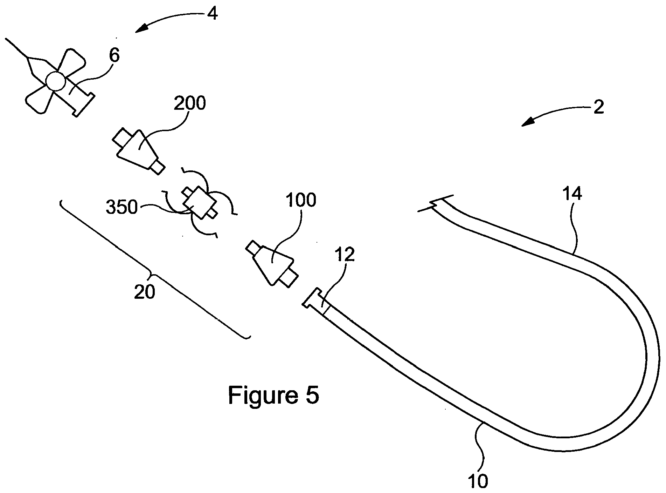

Turning to Figure 4, a second embodiment of the coupling device of the present invention is shown. The coupling device is shown connected to a medical line and an IV catheter assembly in the manner shown in Figure 1. Accordingly, for ease of reference, the components common to this embodiment of Figure 1 and 4 are indicated using the same reference numerals in Figure 4 and reference is made to the foregoing description. The components of the device of Figure 4 are shown in an exploded, disassembled condition in Figure 5.

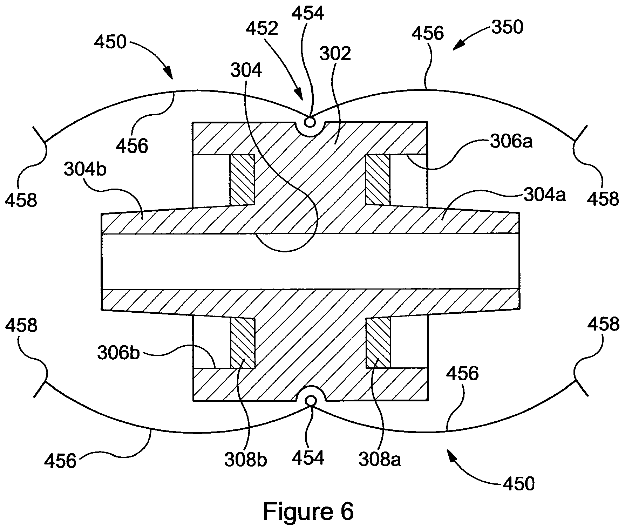

The embodiment of Figure 4 differs from that of Figure 1 by way of the coupling means. In place of the coupling member 400 shown in Figure 1, the embodiment of Figure 4 comprises a plurality of spring clips 450, shown in more detail in Figure 6.

Referring now to Figure 6, there is shown a cross-sectional view of the coupling assembly 350. The coupling assembly 350 is of the same general configuration as that of Figure 3, with common features being indicated using the same reference numerals. In addition, the coupling assembly 350 of this embodiment comprises a plurality of elongate spring clips 450 disposed circumferentially around the body 302. Each spring clip 450 is formed from a resilient material, for example spring steel, and is pivotally mounted at its centre portion 452 to the body 302 by a pin 454. Each portion 456 of the spring clip 450 extending from the central mount is longitudinally curved and terminates in end portion 458 of the spring clip. Each end portion 458 is arranged to releasably engage with the exterior of a respective valve assembly. The arrangement of the portions 456 and the end portions 458 holds the spring clip against its inherent bias, in turn holding both end portions 458 engaged with the respective valve assembly. The form of the spring clip and the form of the engagement of each arm with the respective valve assembly is such that the engagement of the arm with the valve assembly is released upon being subjected to a sufficient force applied longitudinally along the medical line 10. The bias in the spring clip 450 is such that the release of one arm 458 from its valve assembly in turn causes the other arm to release, thus uncoupling the coupling assembly 300 from both valve assemblies.

In use, the components of the device shown in Figure 5 are assembled as shown in Figure 4. In this position, the valves of both valve assemblies 100, 200 are open and fluid can flow between the medical line 10 and the IV catheter assembly 4, in conventional manner. Should a force be applied to the medical line 10, indicated by the arrow D, for example by someone inadvertently pulling on the line, the spring clips 450 are caused to release from engagement with both valve assemblies 100, 200, thereby freeing the coupling assembly 300. The bias, indicated by the arrows E, acting to urge the coupling assembly 300 away from each valve assembly ensures the coupling assembly is fully disengaged from both valve assemblies 100, 200, allowing both valves to close and prevent fluid flow into or out of both the medical line 10 and the IV catheter assembly 4.

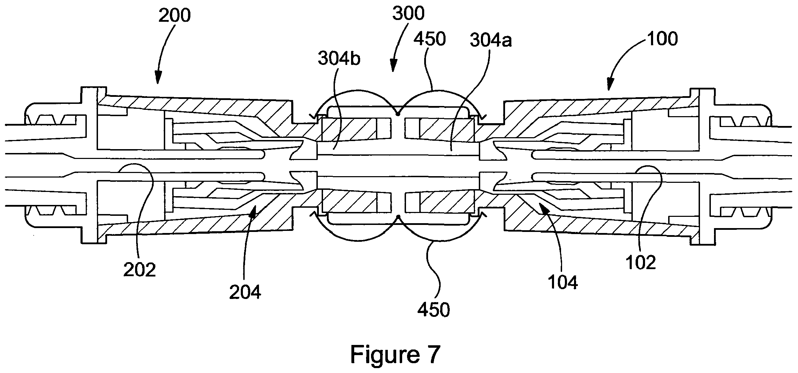

Referring to Figure 7, there is shown a cross-sectional view of a coupling assembly 300 engaged with both a first and second valve assembly 100, 200, as in both Figures 1 and 4. The coupling means shown in Figure 7 comprises spring clips 450. However, other embodiments of the coupling means may be used in an analogous manner. Each valve assembly 100, 200 has a fluid conduit 102, 202 extending therethrough and a Luer activated valve 104, 204 therein of known configuration. Each valve is biased to the closed position. The nozzles 304a, 304b of the coupling assembly 300 engages with the valve and acts as a valve actuator, opening the valve 104, 204 and allowing the flow of fluid through the coupling device. The bias of the valve 104, 204 urges the coupling assembly 300 out of engagement with the valve assembly 100, 200 and away from the valve assembly. The coupling assembly 300 is held in engagement with the valve assemblies 100, 200 by the means described above.

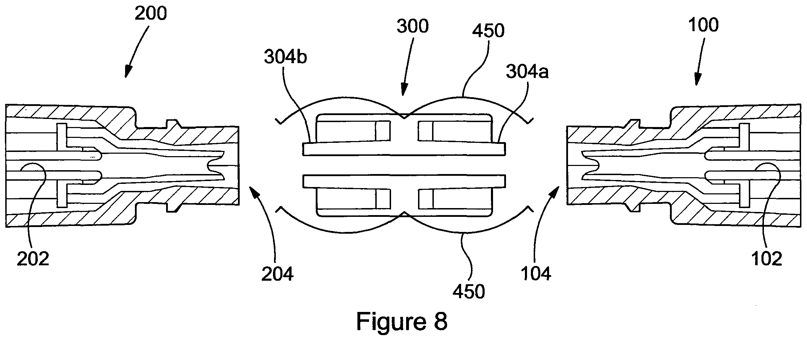

Figure 8 shows a cross-sectional view of the coupling assembly 300 and each valve assembly 100, 200 of Figure 7 in a disengaged condition. As can be seen, with the coupling assembly 300 uncoupled from each valve assembly 100, 200, each valve 104, 204 is closed under the action of its inbuilt bias.

## Claims

1. A coupling assembly (300) for a coupling device (2), the coupling assembly (300) having a conduit (304) therethrough and being for releasably coupling to both of a first valve assembly (100) and a second valve assembly (200), the coupling assembly (300) when so coupled opening both the first and second valves (104, 204) to allow fluid to flow from the first line segment through the coupling assembly (300) to the second line segment or medical device (6);
**characterised in that**
the coupling assembly (300) further comprises releasable coupling means to releasably hold the coupling assembly (300) coupled to both the first and second valve assemblies (100, 200), the coupling means releasing the coupling assembly (300) from both the first and second valve assemblies (100, 200) when the first line segment (10) and the medical device (6) or the first and second line segments (10) are subjected to a sufficiently high separation force;
wherein the coupling means (300) comprises one or more resilient coupling members, the or each coupling member being permanently attached to the coupling assembly (300); wherein the or each coupling member comprises a spring clip (450), wherein the spring clip (450) is permanently attached to the coupling assembly (300) at a central portion (452) and has opposing first and second end portions (458) to releasably engage with a respective one of the first and second valve assemblies (100, 200).

2. The coupling assembly (300) according to claim 1, wherein the coupling assembly (300) comprises first and second valve actuators to open the first and second valves (104, 204) respectively, preferably wherein the first and second valve actuators are disposed at opposite ends of the coupling assembly (300); and/or wherein the actuator is a projection, nozzle or spigot (304a, 304b) extending from the coupling assembly (300); preferably wherein the projection, nozzle or spigot (304a, 304b) is a male Luer taper.

3. The coupling assembly (300) according to either of claims 1 or 2, wherein the coupling assembly (300) is biased out of engagement with at least one of the first and second valve assemblies (100, 200), preferably wherein the coupling assembly (300) is biased out of engagement with both the first and second valve assemblies (100, 200); and/or wherein the bias is provided by the valve (104, 204) in the valve assembly (100, 200); and/or wherein the device further comprises biasing means to bias the coupling assembly (300) away from the at least one valve assembly (100, 200), preferably wherein the biasing means is provided in the coupling assembly (300), more preferably wherein the biasing means is a resilient means (308a, 308b) compressed under the action of the coupling assembly (300) being connected to the valve assembly (100, 200), still more preferably wherein the biasing means is a spring or a portion of resilient material.

4. The coupling assembly (300) according to any preceding claim, wherein the coupling means is a direct action means releasing under the action of a force applied longitudinally along the line (10) and passing longitudinally through the device (2).

5. The coupling assembly (300) according to any preceding claim, wherein the spring clip (450) is pivotally mounted to the coupling assembly (300).

6. The coupling assembly (300) according to any preceding claim, wherein the coupling means is an indirect action means and responds to a force applied to the medical line which is not directed longitudinally through the device, preferably wherein the coupling means comprises a coupling member (400), more preferably wherein the coupling member (400) is flexible; and/or wherein the coupling member (400) is attached to both the first and second valve assemblies (100, 200), preferably wherein the coupling member (400) is permanently attached to one of the first and second valve assemblies (100, 200).

7. The coupling assembly (300) according to claim 6, wherein the coupling member (400) is attachable to a portion of the line (10) spaced from the coupling device (2); preferably wherein the coupling member (400) is indirectly attachable to the said portion of the line (10), or wherein the coupling member (400) is directly attachable to the said portion of the line (10).

8. The coupling assembly (300) according to any preceding claim, wherein the force required to release the coupling means and uncouple the coupling assembly (300) from the first and second valve assemblies (100, 200) is from 0.5 to 20 N.

9. A releasable coupling device (2) for a medical line (10), the device comprising:
a first valve assembly (100) comprising a first valve (104) biased to a closed position, the first valve assembly (104) for attaching to the end portion (12) of a first line segment;
a second valve assembly (200) comprising a second valve (204) biased to a closed position, the second valve assembly (200) for attaching to a medical device (6) or a second line segment; and
a coupling assembly (300) according to any preceding claim.

10. The device (2) according to claim 9, wherein the first and/or the second valve comprises (100, 200) a diaphragm or membrane; preferably wherein the diaphragm or membrane comprises one or more slits therein.

11. The device (2) according to claim 10, wherein the valve (104, 204) comprises a valve actuator to open the valve.

12. The device (2) according to any of claims 9 to 11, wherein the first and/or second valves (104, 204) are activated by the insertion of a projection, nozzle or spigot.

13. The device (2) according to any of claims 9 to 12, further comprising one or more adapters to allow the valve assemblies (100, 200) to be connected to a line (10) or other medical device (6).

14. A kit comprising a coupling device (2) according to any of claims 9 to 13, the kit comprising one or more additional coupling assemblies (300) according to any of claims 1 to 8.

## Patentansprüche

1. Eine Kupplungsbaugruppe (300) für
eine Kupplungsvorrichtung (2), wobei die Kupplungsbaugruppe (300) ein dadurch durchgehendes Rohr (304) aufweist und zum lösbaren Koppeln sowohl mit einer ersten Ventilbaugruppe (100) als auch einer zweiten Ventilbaugruppe (200) dient, wobei die Kupplungsbaugruppe (300), wenn sie so gekoppelt ist, sowohl das erste als auch das zweite Ventil (104, 204) öffnet, um zu ermöglichen, dass Flüssigkeit von dem ersten Leitungssegment durch die Kupplungsbaugruppe (300) zu dem zweiten Leitungssegment oder der medizinischen Vorrichtung (6) fließt;
**dadurch gekennzeichnet, dass**
die Kupplungsbaugruppe (300) ferner lösbare Kupplungsmittel umfasst, um die Kupplungsbaugruppe (300) mit der ersten und der zweiten Ventilbaugruppen (100, 200) lösbar gekoppelt zu halten, wobei die Kupplungsmittel die Kupplungsbaugruppe (300) sowohl von der ersten als auch von der zweiten Ventilbaugruppen (100, 200) lösen, wenn das erste Leitungssegment (10) und die medizinische Vorrichtung (6) oder das erste und zweite Leitungssegment (10) einer ausreichend hohen Trennkraft ausgesetzt sind;
wobei das Kupplungsmittel (300) ein oder mehrere federnde Kupplungselemente umfasst, wobei das oder jedes Kupplungselement permanent an der Kupplungsbaugruppe (300) befestigt ist; wobei das oder jedes Kupplungselement eine Federklammer (450) umfasst, wobei die Federklammer (450) an einem zentralen Abschnitt (452) permanent an der Kupplungsbaugruppe (300) befestigt ist und gegenüberliegende erste und zweite Endabschnitte (458) aufweist, die lösbar mit jeweils der ersten oder der zweiten Ventilbaugruppe (100, 200) in Eingriff stehen.

2. Kupplungsbaugruppe (300) nach Anspruch 1, wobei die Kupplungsbaugruppe (300) einen ersten und einen zweiten Ventilantrieb jeweils zum Öffnen des ersten und des zweiten Ventils (104, 204) umfasst, wobei der erste und der zweite Ventilantriebe vorzugsweise an entgegengesetzten Enden der Kupplungsbaugruppe (300) angeordnet sind; und/oder wobei der Antrieb ein Vorsprung, eine Düse oder ein Stutzen (304a, 304b) ist, der sich von der Kupplungsbaugruppe (300) erstreckt; wobei der Vorsprung vorzugsweise die Düse oder der Stutzen (304a, 304b) ein männlicher Luer-Konus ist.

3. Kupplungsbaugruppe (300) nach einem der Ansprüche 1 oder 2, wobei die Kupplungsbaugruppe (300) vorzugsweise aus dem Eingriff mit wenigstens der ersten oder der zweiten Ventilbaugruppe (100, 200) vorgespannt ist, wobei die Kupplungsbaugruppe (300) aus dem Eingriff mit sowohl der ersten als auch der zweiten Ventilbaugruppe (100, 200) vorgespannt ist; und/oder wobei die Vorspannung durch das Ventil (104, 204) in der Ventilbaugruppe (100, 200) bereitgestellt wird; und/oder wobei die Vorrichtung ferner Vorspannmittel umfasst, um die Kupplungsbaugruppe (300) von der wenigstens einen Ventilbaugruppe (100, 200) weg vorzuspannen, wobei das Vorspannmittel vorzugsweise in der Kupplungsbaugruppe (300) vorgesehen ist, das Vorspannmittel bevorzugter ein federndes Mittel (308a, 308b) ist, das unter der Wirkung der Kupplungsbaugruppe (300) zusammengedrückt wird, die mit der Ventilbaugruppe (100, 200) verbunden ist, wobei das Vorspannmittel noch bevorzugter eine Feder oder ein Abschnitt eines federnden Materials ist.

4. Kupplungsbaugruppe (300) nach einem der vorhergehenden Ansprüche, wobei das Kupplungsmittel ein direkt wirkendes Mittel ist, das sich unter der Wirkung einer Kraft löst, die in Längsrichtung entlang der Linie (10) ausgeübt wird und in Längsrichtung durch die Vorrichtung (2) verläuft.

5. Kupplungsbaugruppe (300) nach einem der vorhergehenden Ansprüche, wobei die Federklammer (450) an der Kupplungsbaugruppe (300) schwenkbar angebracht ist.

6. Kupplungsbaugruppe (300) nach einem der vorhergehenden Ansprüche, wobei das Kupplungsmittel ein indirekt wirkendes Mittel ist und auf eine auf die medizinische Leitung ausgeübte Kraft reagiert, die nicht in Längsrichtung durch die Vorrichtung gerichtet ist, wobei das Kupplungsmittel vorzugsweise ein Kupplungselement (400) aufweist, wobei das Kupplungselement (400) bevorzugter flexibel ist; und/oder wobei das Kupplungselement (400) sowohl an der ersten als auch an der zweiten Ventilbaugruppe (100, 200) angebracht ist, wobei das Kupplungselement (400) vorzugsweise permanent an der ersten oder der zweiten Ventilbaugruppe (100, 200) befestigt ist.

7. Kupplungsbaugruppe (300) nach Anspruch 6, wobei das Kupplungselement (400) an einem Abschnitt der Leitung (10) beanstandet von der Kupplungsvorrichtung (2) angebracht werden kann; wobei das Kupplungselement (400) vorzugsweise indirekt an dem Abschnitt der Leitung (10) angebracht werden kann, oder wobei das Kupplungselement (400) direkt an dem Abschnitt der Leitung (10) angebracht werden kann.

8. Kupplungsbaugruppe (300) nach einem der vorhergehenden Ansprüche, wobei die Kraft, die erforderlich ist, um die Kupplungsmittel zu lösen und die Kupplungsbaugruppe (300) von der ersten und der zweiten Ventilbaugruppe (100, 200) zu entkoppeln zwischen 0,5 und 20 N liegt.

9. Eine lösbare Kupplungsvorrichtung (2) für eine medizinische Leitung (10), wobei die Vorrichtung Folgendes umfasst:
eine erste Ventilbaugruppe (100) mit einem ersten Ventil (104), das in eine geschlossene Position vorgespannt ist, wobei die erste Ventilbaugruppe (104) zum Anbringen an dem Endabschnitt (12) eines ersten Leitungssegments vorgesehen ist;
eine zweite Ventilbaugruppe (200) mit einem zweiten Ventil (204), das in eine geschlossene Position vorgespannt ist, die zweite Ventilbaugruppe (200) zum Anbringen an einer medizinischen Vorrichtung (6) oder einem zweiten Leitungssegment; und
eine Kupplungsbaugruppe (300) nach einem der vorhergehenden Ansprüche.

10. Vorrichtung (2) nach Anspruch 9, wobei das erste und/oder die zweite Ventil (100, 200) eine Diaphragma oder Membran umfasst; wobei die Diaphragma oder Membran vorzugsweise einen oder mehrere Schlitze umfasst.

11. Vorrichtung (2) nach Anspruch 10, wobei das Ventil (104, 204) einen Ventilantrieb zum Öffnen des Ventils umfasst.

12. Vorrichtung (2) nach einem der Ansprüche 9 bis 11, wobei das erste und/oder das zweite Ventil (104, 204) durch Einsetzen eines Vorsprungs, einer Düse oder eines Stutzens aktiviert werden.

13. Vorrichtung (2) nach einem der Ansprüche 9 bis 12, ferner umfassend einen oder mehrere Adapter, um es den Ventilbaugruppen (100, 200) zu ermöglichen, an eine Leitung (10) oder eine andere medizinische Vorrichtung (6) angeschlossen zu werden.

14. Kit, umfassend eine Kupplungsvorrichtung (2) nach einem der Ansprüche 9 bis 13, wobei das Kit eine oder mehrere zusätzliche Kupplungsbaugruppen (300) nach einem der Ansprüche 1 bis 8 umfasst.

## Revendications

1. Dispositif d'accouplement (300) pour
un dispositif d'accouplement (2), le dispositif d'accouplement (300) comportant un conduit (304) le traversant et étant destiné à un accouplement amovible à un premier ensemble de valve (100) et un second ensemble de valve (200), le dispositif d'accouplement (300) ouvrant ainsi les première et seconde valves (104, 204) pour laisser le fluide passer de la première section de ligne à la deuxième section de ligne ou dispositif médical (6) en traversant le dispositif d'accouplement (300) ;
**caractérisé en ce que**
le dispositif d'accouplement (300) comprend en outre des moyens d'accouplement détachables pour maintenir de manière détachable le dispositif d'accouplement (300) couplé aux premier et second ensembles de valve (100, 200), les moyens d'accouplement libérant le dispositif d'accouplement (300) des premier et second ensembles de valve (100, 200) lorsque la première section de ligne (10) et le dispositif médical (6) ou les premières et deuxième sections de ligne (10) sont soumis à une force de séparation suffisamment élevée ;
dans lequel le moyen d'accouplement (300) comprend un ou plusieurs éléments d'accouplement élastiques, le ou chaque élément d'accouplement étant fixé de façon permanente au dispositif d'accouplement (300) ; dans lequel le ou chaque élément d'accouplement comprend un clip élastique (450), dans lequel le clip élastique (450) est fixé de façon permanente au dispositif d'accouplement (300) à une partie centrale (452) et a une première et une deuxième parties terminales opposées (458) pour s'engager de manière amovible avec l'un des premier et second ensembles de valve respectivement (100, 200).

2. Ensemble d'accouplement (300) selon la revendication 1, dans lequel le dispositif d'accouplement (300) comprend des premier et second actionneurs de valve pour ouvrir respectivement les première et seconde valves (104, 204), de préférence dans lequel les premier et second actionneurs de valve sont disposés aux extrémités opposées du dispositif d'accouplement (300) ; et/ou dans lequel l'actionneur est une saillie, une buse ou un embout (304a, 304b) s'étendant à partir du dispositif d'accouplement (300) ; de préférence dans lequel la saillie, la buse ou l'embout (304a, 304b) est un cône Luer mâle.

3. Dispositif d'accouplement (300) selon l'une des revendications 1 ou 2, dans lequel le dispositif d'accouplement (300) est polarisé hors de l'engagement avec au moins l'un des premier et second ensembles de valve (100, 200), de préférence dans lequel le dispositif d'accouplement (300) est polarisé hors de l'engagement avec les premier et second ensembles de valve (100, 200) ; et/ou dans laquelle la polarisation est fournie par la valve (104, 204) dans l'ensemble de valve (100, 200) ; et/ou dans laquelle le dispositif comprend en outre un moyen de polarisation pour polariser le dispositif d'accouplement (300) en l'éloignant d'au moins un ensemble de valve (100, 200), de préférence dans lequel le moyen de précontrainte est prévu dans le dispositif d'accouplement (300), plus préférentiellement dans lequel le moyen de précontrainte est un moyen élastique (308a, 308b) comprimé sous l'action du dispositif d'accouplement (300) étant connecté à l'ensemble de valve (100, 200), encore plus préférablement dans lequel le moyen de précontrainte est un ressort ou une partie de matériau élastique.

4. Dispositif d'accouplement (300) selon l'une quelconque des revendications précédentes, dans lequel le moyen d'accouplement est un moyen d'action directe libérant sous l'action d'une force appliquée longitudinalement le long de la ligne (10) et traversant longitudinalement le dispositif (2).

5. Dispositif d'accouplement (300) selon l'une quelconque des revendications précédentes, dans lequel la pince à ressort (450) est montée de manière pivotante sur le dispositif d'accouplement (300).

6. Un ensemble d'accouplement (300) selon l'une quelconque des revendications précédentes, dans lequel le moyen d'accouplement est un moyen à action indirecte et répond à une force appliquée à la ligne médicale qui n'est pas dirigée longitudinalement à travers le dispositif, de préférence dans laquelle le moyen d'accouplement comprend un élément d'accouplement (400), plus préférablement dans laquelle l'élément d'accouplement (400) est flexible ; et/ou dans lequel l'élément d'accouplement (400) est fixé à la fois aux premier et second ensembles de valve (100, 200), de préférence dans lequel l'élément d'accouplement (400) est fixé de façon permanente à l'un des premier et deuxième ensembles de valve (100, 200).

7. Dispositif d'accouplement (300) selon la revendication 6, dans lequel l'élément d'accouplement (400) peut être fixé à une partie de la ligne (10) espacée du dispositif d'accouplement (2) ; de préférence dans lequel l'élément d'accouplement (400) peut être fixé indirectement à ladite partie de la ligne (10), ou dans lequel l'élément d'accouplement (400) peut être fixé directement à ladite partie de la ligne (10).

8. Un ensemble d'accouplement (300) selon l'une quelconque des revendications précédentes, dans lequel la force requise pour libérer le moyen d'accouplement et désaccoupler le dispositif d'accouplement (300) des premier et second ensembles de valve (100, 200) est de 0,5 à 20 N.

9. Dispositif d'accouplement amovible (2) pour une ligne médicale (10), le dispositif comprenant :
un premier ensemble de valve (100) comprenant une première valve (104) polarisée vers une position fermée, le premier ensemble de valve (104) destiné à être fixé à la partie d'extrémité (12) d'un premier segment de ligne ;
un deuxième ensemble de valve (200) comprenant une deuxième valve (204) polarisée en position fermée, le deuxième ensemble de valve (200) destiné à être fixé à un dispositif médical (6) ou à un segment de seconde ligne ; et
un ensemble d'accouplement (300) selon l'une quelconque des revendications précédentes.

10. Dispositif (2) selon la revendication 9, dans lequel la première et/ou la seconde valve comprend (100, 200) un diaphragme ou une membrane ; dans laquelle le diaphragme ou la membrane comprend de préférence une ou plusieurs fentes.

11. Dispositif (2) selon la revendication 10, dans lequel la valve (104, 204) comprend un actionneur de valve pour ouvrir la valve.

12. Dispositif (2) selon l'une quelconque des revendications 9 à 11, dans lequel les première et/ou seconde valves (104, 204) sont activées par l'insertion d'une saillie, d'une buse ou d'un embout.

13. Dispositif (2) selon l'une quelconque des revendications 9 à 12, comprenant en outre un ou plusieurs adaptateurs pour permettre aux ensembles de valves (100, 200) d'être connectés à une ligne (10) ou à un autre dispositif médical (6).

14. Kit comprenant un dispositif d'accouplement (2) selon l'une quelconque des revendications 9 à 13, le kit comprenant un ou plusieurs ensembles d'accouplement supplémentaires (300) selon l'une quelconque des revendications 1 à 8.
